# EUROPÄISCHE PATENTSCHRIFT

(11) **EP 2 422 726 B1**
(45) Veröffentlichungstag und Bekanntmachung des Hinweises auf die Patenterteilung: **10.04.2013**
(21) Anmeldenummer: 11178452.6
(22) Anmeldetag: 23.08.2011
(51) Int. Cl.: A61B 17/56, A61B 17/70, A61M 29/02, A61B 17/88

(54) **Vorrichtungen zur Ballon-Wirbelkyphoplastie**
Devices for balloon spine kyphoplasty
Dispositifs de cyphoplastie vertébrale à ballonnet

(30) Priorität: 23.08.2010 RU 2010134840
(43) Veröffentlichungstag der Anmeldung: 29.02.2012
(73) Patentinhaber: Korsakov, Konstantin Vladimirovich, Moskau 125167 (RU); Vilkov, Dmitriy Yuryevich, Samara 443087 (RU); Dubrovin, Andrey Eugenyevich, Moskovskaya oblast 142771 (RU); Toma, Alexander Ilyich, Saratov 410056 (RU)
(72) Erfinder: Toma, Alexander Ilyich, 410056 Saratov (RU); Elkin, Vladimir Alexandrovich, 410031 Saratov (RU); Norkin, Alexey Igorevich, 410031 Saratov (RU); Vilkov, Dmitriy Yuryevich, 410002 Saratov (RU); Toma, Ilya Alexandrovich, 410056 Saratov (RU); Yankin, Sergey Sergeyevich, 410039 Saratov (RU)
(74) Vertreter: Sloboshanin, Sergej

(56) Entgegenhaltungen:
- EP-A1- 2 106 820
- WO-A1-2010/063111
- WO-A2-2006/116760
- WO-A2-2007/067726

## Beschreibung

Die vorliegende Erfindung betrifft Vorrichtungen zur Ballon-Wirbelkyphoplastie gemäß den Oberbegriffen der Ansprüche 1 und 9.

Die Erfindung betrifft die Medizintechnik, insbesondere Instrumente für die Traumatologie, Orthopädie, Neurochirurgie und Vertebrologie, und kann zur Rekonstruktion der Wirbelsäule bei einem chirurgischen Eingriffsverfahren mit Ballon-Kyphoplastie verwendet werden, das auf der Grundlage der Punktions-Vertebroplastie zur Behandlung von Patienten mit Kompressionsbrüchen oder Kompressions-Splitterbrüchen der Wirbelkörper infolge von Osteoporose oder traumatischen Schädigungen entwickelt wurde und das es ermöglicht, die infolge der kyphotischen Verformung verlorene Höhe des Wirbelkörpers wiederherzustellen und sie mit Hilfe von in den Wirbelkörper injiziertem Knochenersatzmaterial zu verstärken [E.G. Pedachenko, S.V. Kushchaev: Punktsionnaya vertebroplastika, A.L.D., Kiew 2005, S. 25].

Es sind Lösungen bekannt, die in dem Patent RU 2351375 und dem Gebrauchsmuster RU 71534 derselben Anmelderinnen sowie in den Patenten US 5972015 und US 6899719 vorgestellt wurden. Darin sind Vorrichtungen zur Ballon-Wirbelkyphoplastie beschrieben, die aus einer tragenden Katheterröhre mit einem am distalen Ende befestigten Dilatationsballon aus elastischem Material bestehen. Zur Korrektur der kyphotischen Deformation ist der Dilatationsballon so ausgeführt, dass er sich unter dem Einfluss einer Druckflüssigkeit in dem Wirbelkörper ausdehnen kann. Zur Injektion des Knochenersatzmaterials wird ein Trokarinjektor verwendet.

Der nächstgelegene Stand der Technik für die erste Variante der Erfindung ist eine Vorrichtung zur Ballon-Wirbelkyphoplastie [Patentanmeldung US 2010152654], die aus einer tragenden Katheterröhre mit einem am distalen Ende befestigten Dilatationsballon aus elastischem Material besteht. An dem Dilatationsballon ist ein Element befestigt, das es ermöglicht, eine thermische, elektromagnetische oder optische Wirkung auf die Gewebe des Organismus des Patienten auszuüben. Zum Injizieren des Knochenersatzmaterials wird ein Trokarinjektor verwendet.

Der nächstgelegene Stand der Technik für die zweite Variante der Erfindung ist eine andere Vorrichtung, die in der oben genannten Anmeldung beschrieben ist und die aus einer tragenden Katheterröhre mit einem am distalen Ende befestigten Dilatationsballon aus elastischem Material besteht. In den proximalen Teil des Dilatationsballons ist ein karkassenartiger Ausdehnungsbegrenzer eingefügt. Zum Injizieren des Knochenersatzmaterials wird ein Trokarinjektor verwendet.

Jedoch ist dabei die Möglichkeit einer unzureichenden Wiederherstellung der Höhe des Wirbelkörpers nicht auszuschließen. Dies ist durch die Unmöglichkeit einer weiteren Ausdehnung des Ballons und einer weiteren Verdichtung des schwammigen Gewebes an seiner Peripherie bei vorgegebenem Flüssigkeitsdruck bedingt.

Aufgabe der Erfindung ist die Entwicklung der Konstruktion einer Vorrichtung, die es ermöglicht, die größtmögliche Wiederherstellung der Höhe des geschädigten Wirbelkörpers zu erzielen.

Der Kern der ersten Variante der Erfindung liegt darin, dass eine Vorrichtung zur Ballon-Wirbelkyphoplastie, die eine Einrichtung zur Injektion von Knochenersatzmaterial in den Wirbelkörper und eine tragende Katheterröhre mit einem Kanal und einer damit verbundenen Druckflüssigkeitspumpe in einen am distalen Ende der tragenden Katheterröhre abdichtend befestigten Dilatationsballon aus elastischem, dehnbarem Material aufweist, einen Elektromagnet umfasst, der einen Ausgang zur einer Wechselstromquelle aufweist und so ausgebildet ist, dass er am Körper des Patienten entlang der Wirbelsäule angeordnet werden kann, wobei am distalen Teil des Dilatationsballons ferromagnetische Elemente eingefügt sind, wobei der Elektromagnet so ausgebildet ist, dass er ein Wechselmagnetfeld erzeugen und damit auf die ferromagnetischen Elemente einwirken kann, um stoß-vibrierende Übertragungsbewegungen am distalen Teil des Dilatationsballons zu ermöglichen.

Außerdem wird eine Vorrichtung zur Ballon-Wirbelkyphoplastie mit den oben genannten Merkmalen angemeldet, bei der der Elektromagnet eine langgestreckte zylindrische Form hat.

Außerdem wird eine Vorrichtung zur Ballon-Wirbelkyphoplastie mit den oben genannten Merkmalen angemeldet, bei der die ferromagnetischen Elemente in Form von Teilchen ausgebildet sind, die in die Wände des distalen Teils des Dilatationsballons eingefügt sind. Außerdem wird eine Vorrichtung zur Ballon-Wirbelkyphoplastie mit den oben genannten Merkmalen angemeldet, bei der die ferromagnetischen Elemente in Form von Fäden ausgebildet sind, die an der Innenseite des distalen Teils des Dilatationsballons angeordnet sind.

Es wird weiterhin eine Vorrichtung zur Ballon-Wirbelkyphoplastie mit den oben genannten Merkmalen angemeldet, bei der der Dilatationsballon zusätzlich eine und/oder mehrere Kammern aufweist.

Der Kern der zweiten Variante der Erfindung liegt darin, dass bei der Vorrichtung zur Ballon-Wirbelkyphoplastie, die eine Einrichtung zur Injektion des Knochenersatzmaterials in den Wirbelkörper, eine tragende Katheterröhre mit einem Kanal und einer damit verbundenen Druckflüssigkeitspumpe in einen am distalen Ende der tragenden Katheterröhre abdichtend befestigten Dilatationsballon aus elastischem, dehnbarem Material sowie einen in den proximalen Teil des Dilatationsballons eingefügten karkassenartigen Begrenzer für die Ausdehnung des Ballons aus nicht dehnbarem Material umfasst, in die Flüssigkeitspumpe ein Hydroimpulsmodulator eingefügt ist, der Ausgänge zu einem Elektroimpulsgenerator aufweist, wobei der Hydroimpulsmodulator zur Erzeugung von Hydroimpulsen, die auf den Dilatationsballon einwirken, und zur Erzeugung von stoß-vibrierenden Übertragungsbewegungen in dessen distalem Teil ausgebildet ist.

Außerdem wird eine Vorrichtung zur Ballon-Wirbelkyphoplastie mit den oben genannten Merkmalen angemeldet, bei der der Hydroimpulsmodulator aus einem von der distalen Seite mit einer flexiblen Membran aus ferromagnetischem Material abdichtend befestigten Elektromagneten besteht, der eine Wicklung und einen verstärkten Kern umfasst, der die Wicklung vollständig umschließt.

Das technische Ergebnis der Erfindung liegt in dem Versuch der Verwirklichung zusätzlicher Möglichkeiten bei der Durchführung der Behandlung von Kompressionsbrüchen der Wirbelkörper durch Einführung in eine Vorrichtung zur Ballon-Wirbelkyphoplastie einer Einheit, die durch weiche stoß-vibrierende Übertragungsbewegungen des distalen Teils des Dilatationsballons, die eine Überwindung der Kompression ermöglichen, die die vollständige Wiederherstellung der Höhe des Wirbelkörpers behindert, und die dessen Korrektur sowie ein dichteres "Packen" der Splitter des Wirbels um den ausgebildeten Raum ermöglichen, eine sanfte Einwirkung auf das schwammige Knochengewebe hat. Die Verwendung der in dieser Anmeldung vorgeschlagenen Verbesserungen für die Ballon-Kyphoplastie ermöglicht die Verwirklichung von gesteuerten und gezielten Einwirkungen auf den Wirbelkörper, was das Erzielen der größtmöglichen Wiederherstellung der Höhe des geschädigten Wirbelkörpers ermöglicht.

Die Verwendung des Dilatationsballons, der zusätzlich eine und/oder mehrere Kammern aufweist, ermöglicht eine Erhöhung der Wirksamkeit bei der schrittweisen Wiederherstellung der Höhe des geschädigten Wirbelkörpers, wobei die unteren Kammern eine Art "Sockel" für die oberen Kammern bilden.

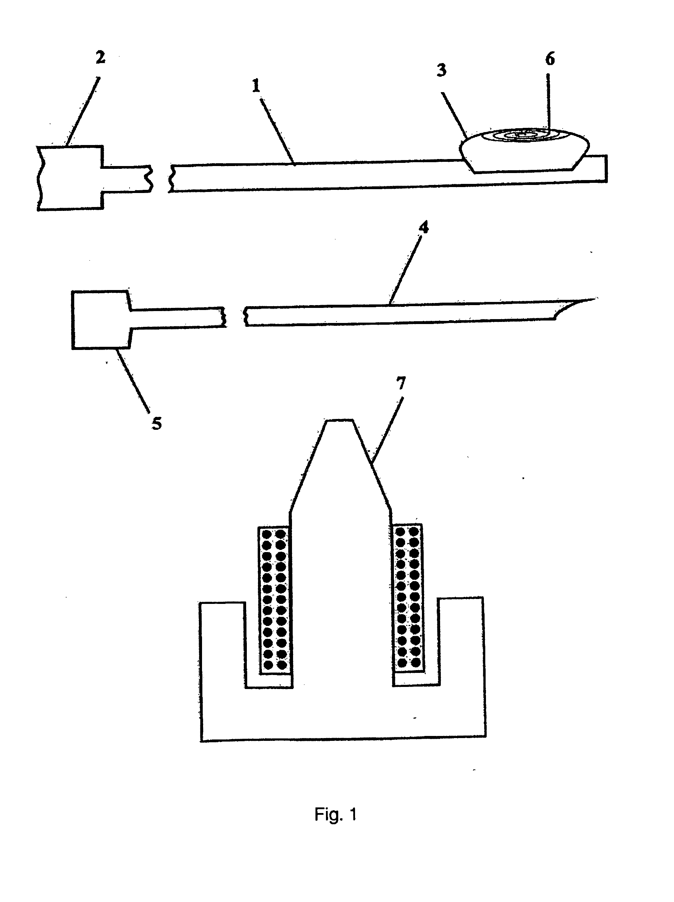

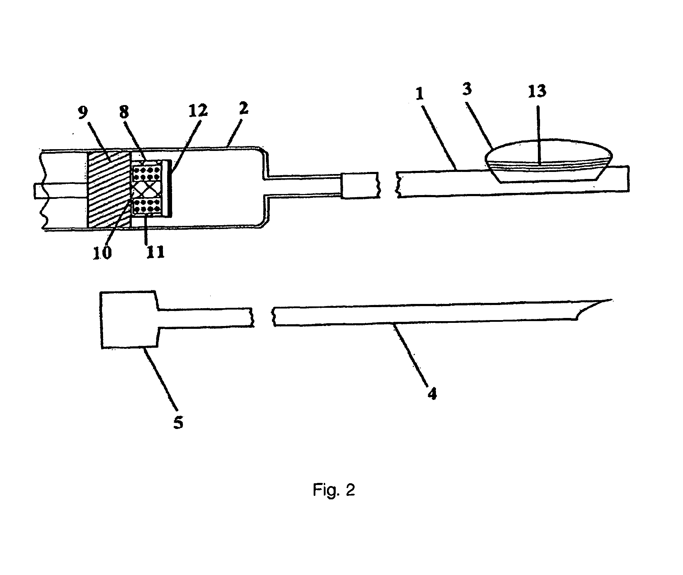

Die Erfindung wird mit Hilfe von Fig. 1 bis 6 erläutert, die Folgendes darstellen:
- Fig. 1:: die Vorrichtung zur Ballon-Wirbelkyphoplastie (Variante 1),
- Fig. 2:: die Vorrichtung zur Ballon-Wirbelkyphoplastie (Variante 2),
worin mit den Bezugszeichen 1 bis 13 bezeichnet sind:
1 die tragende Katheterröhre,
2 die Pumpe,
3 der Dilatationsballon,
4 die Nadel,
5 die Spritze mit dem Knochenersatzmaterial,
6 die ferromagnetischen Elemente,
7 der Elektromagnet,
8 der Hydroimpulsmodulator,
9 der Kolben der Pumpe,
10 der Kern,
11 die Wicklung,
12 die Membran,
13 der karkassenartige Ausdehnungsbegrenzer.

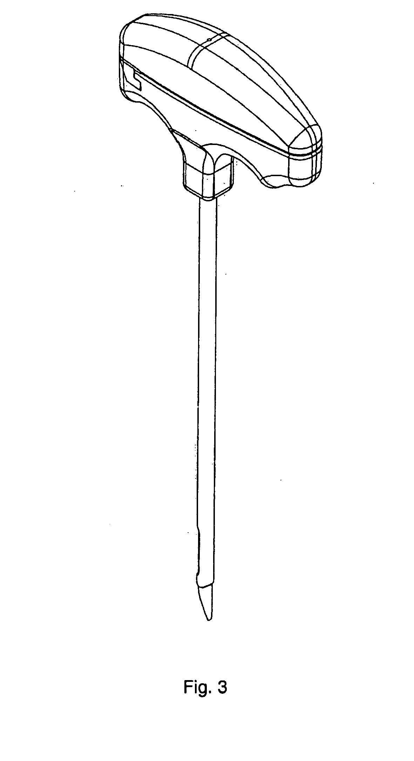

- Fig. 3:: der Trokar und der Mandrin im zusammengesetzten Zustand;

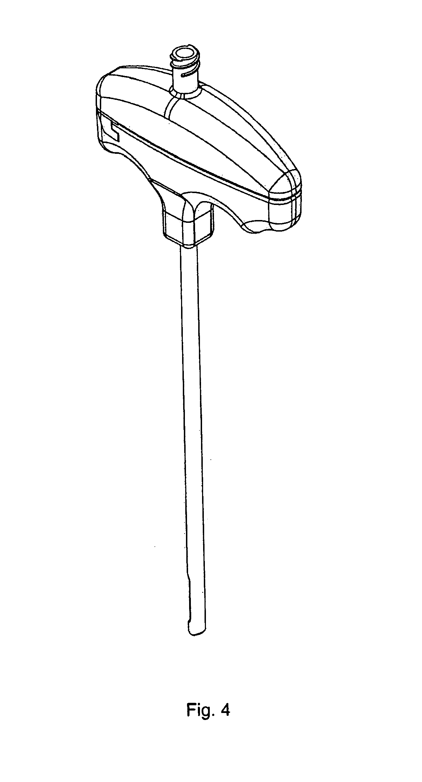

- Fig. 4:: der Trokar und die tragende Katheterröhre mit dem Dilatationsballon im zusammengesetzten Zustand;

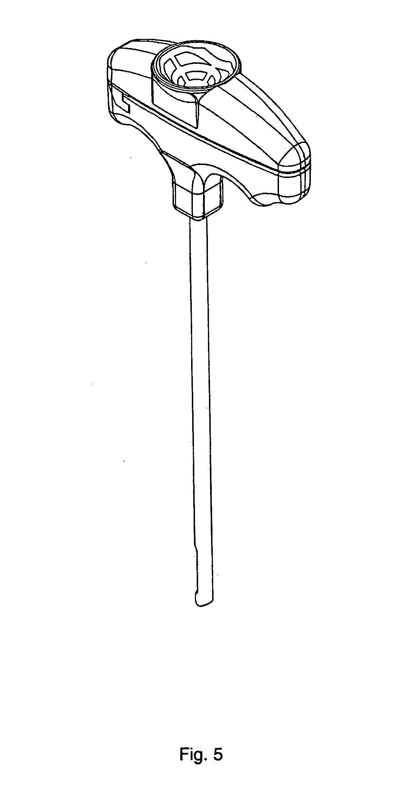

- Fig. 5:: der Trokar und das Basisteil mit dem Trichter im zusammengesetzten Zustand;

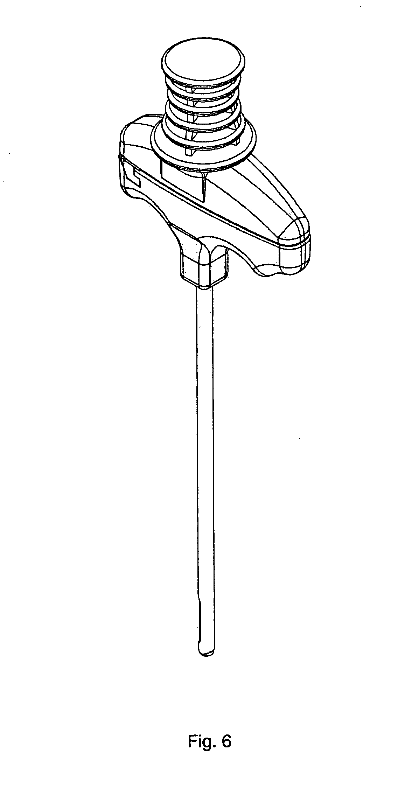

- Fig. 6:: der Trokar, das Basisteil mit dem Trichter und dem Schaftstößel im zusammengesetzten Zustand.

Bei beiden Varianten umfasst die Vorrichtung zur Ballon-Wirbelkyphoplastie eine tragende Katheterröhre 1 mit einem Kanal, an den die Druckflüssigkeitspumpe 2 angeschlossen ist. Am distalen Ende der tragenden Katheterröhre 1 ist der Dilatationsballon 3 abdichtend befestigt.

Als Dilatationsballon können Ballons aus Latex, Silikon oder einem beliebigen anderen elastischen, dehnbaren Material mit einem spezifischen Berstdruck von wenigstens 20 atm verwendet werden.

Die Vorrichtung kann ferner einen Trokar, einen Mandrin und eine Einrichtung zum Einführen von granuliertem Knochenersatzmaterial umfassen. Die Letztgenannte besteht aus einem Basisteil mit einem Trichter und einem Schaftstößel mit Handgriff. Die tragende Katheterröhre und der Mandrin sind koaxial gestreckt innerhalb und entlang des Trokars ausgeführt, wobei sie nacheinander in ihn hinein- und aus ihm herausgeführt werden können.

Das Basisteil mit dem Trichter der Einrichtung zum Einführen von granuliertem Knochenersatzmaterial, der Mandrin von seiner proximalen Kante her und die tragende Katheterröhre sind im Bereich der Verbindung mit der Flüssigkeitspumpe an die Form des Trokarhandgriffs als Paare Vorsprung-Öffnung angepasst, wobei sie dicht mit einander verbunden werden können.

Bei beiden Varianten umfasst die Vorrichtung zur Ballon-Wirbelkyphoplastie eine Einrichtung zur Injektion des Knochenersatzmaterials in den Wirbelkörper, die aus einer Nadel 4 und einer Spritze mit dem Knochenersatzmaterial 5 besteht.

In die Konstruktion der Vorrichtung zur Ballon-Kyphoplastie ist eine Einheit zur Erzeugung von stoß-vibrierenden Übertragungsbewegungen des distalen Teils des Dilatationsballons eingefügt.

Bei der ersten Variante ist diese Einheit in Form ferromagnetischer Elemente 6 und eines Elektromagneten 7, die in den distalen Teil des Dilatationsballons 3 eingefügt sind, vorgesehen. Die ferromagnetischen Elemente 6 sind in den distalen Teil des Dilatationsballons 3 eingefügt und können in Form von gleichmäßig verteilten Magnetteilchenelementen mit Abmessungen von 100 bis 200 *µ*m oder Fäden mit einem Durchmesser von 50 bis 100 *µ*m aus ferromagnetischem Material ausgebildet sein, die beispielsweise in Form einer Spirale angeordnet und an der Innenseite des distalen Teils des Dilatationsballons befestigt sind. Der Elektromagnet 7 ist in Form eines Kerns mit einer Wicklung ausgebildet und weist Ausgänge zu einer Wechselstromquelle auf. Der Elektromagnet 7 kann eine langgestreckte Form haben.

Bei der zweiten Variante ist diese Einheit in Form eines Hydroimpulsmodulators 8 ausgebildet, der in die Flüssigkeitspumpe 2 eingefügt ist. Der Hydroimpulsmodulator 8 umfasst einen Elektromagnet in Form eines "topfförmigen" Kerns 10 mit einer Wicklung 11, der an dem Kolben 9 der Flüssigkeitspumpe angeordnet ist. Der Kern 10 ist als verstärkter Typ ausgebildet, er umschließt die Wicklung 11 vollständig. An der distalen Seite des Elektromagneten ist eine Membran 12 befestigt, deren Steifheit den Widerstand gegen Wasserdrücke im Bereich von 10 atm im Inneren des Hohlraums der Pumpe 2 ermöglicht. Die Membran 12 ist aus ferromagnetischem Material hergestellt und dichtet die Wicklung 11 ab, auf diese Weise wird ein wasserundurchlässiger Aufbau hergestellt. Der Spalt zwischen dem Kern 10 und der Membran 12 liegt im Bereich von 1 bis 1,5 mm. In den Dilatationsballon 3 ist in seinem proximalen Teil ein karkassenartiger Ausdehnungsbegrenzer 13 aus nicht elastischem Material eingefügt.

Bei der ersten Variante der angemeldeten Vorrichtung ist die Verwendung eines Dilatationsballons 3 möglich, der zusätzlich eine und/oder mehrere Kammern aufweist, was eine Erhöhung der Wirksamkeit bei der schrittweisen Wiederherstellung der Höhe des geschädigten Wirbelkörpers ermöglicht, wobei die unteren Kammern eine Art "Sockel" für die oberen Kammern bilden.

Die Vorrichtung funktioniert wie folgt.

Der Patient wird in Bauchlage auf den Operationstisch gelegt. Der Operationsbereich wird markiert, wo Orientierungspunkte zur Durchführung einer transpedikulären Punktion des geschädigten Wirbels angeordnet werden, die mittels Einführung eines Röhrenleiters in Form eines Trokars mit einem Mandrin zur Einführung der angemeldeten Vorrichtung durchgeführt wird, kontrolliert durch Röntgen und/oder Computertomografie und unter Lokalanästhesie. Der Mandrin wird aus dem Trokar entfernt und durch diesen wird durch den Röhrenleiter in dem in dem Wirbelkörper ausgebildeten Kanal der distale Teil der tragenden Katheterröhre 1 installiert, an der am distalen Ende der Dilatationsballon 3, der Röntgenkontrastmittel aufweist, abdichtend, beispielsweise durch abdichtende Nähte, befestigt wird. Mit Hilfe der Sichtkontrolle wird die endgültige Anordnung des Dilatationsballons 3 im geschädigten Wirbelkörper bestimmt.

Anschließend wird die Höhe des Wirbelkörpers wiederhergestellt. Hierfür wird mit Hilfe der Pumpe 2 der Dilatationsballon unter Druck durch die Flüssigkeit ausgedehnt. Entsprechend der Ausdehnung wird die Form des geschädigten Wirbelkörpers gerichtet, die kyphotische Verformung wird korrigiert und das schwammige Knochengewebe wird verdichtet und die Knochensplitter werden gerichtet, wodurch ein Hohlraum gebildet wird.

Bei unvollständiger Wiederherstellung der Höhe des Wirbelkörpers wird zusätzlich mittels Übertragung der stoß-vibrierenden Übertragungsbewegungen des distalen Teils des Dilatationsballons 3 mit Hilfe der Elemente der oben genannten Einheit, die in den Aufbau der Vorrichtung eingefügt sind, vorsichtig auf das schwammige Knochengewebe eingewirkt.

Hierfür wird bei der ersten Variante der Vorrichtung der Elektromagnet 7 an eine Wechselstromquelle angeschlossen. Im Moment des Einschaltens wird er am Körper des Patienten entlang der Wirbelsäule angeordnet, so dass die Symmetrieachse des Elektromagneten auf den distalen Teil des Dilatationsballons 3 gerichtet ist. Der Elektromagnet 7 erzeugt ein Wechselmagnetfeld, das auf die ferromagnetischen Elemente 6 einwirkt, indem es sie periodisch anzieht, wobei der distale Teil des Dilatationsballons stoß-vibrierende Übertragungsbewegungen ausführt, die vorsichtig auf das schwammige Knochengewebe im Inneren des geschädigten Wirbelkörpers einwirken.

Bei der zweiten Variante der Erfindung ist der Hydroimpulsmodulator 8 an einen Elektroimpulsgenerator angeschlossen. Mit seiner Hilfe werden Magnetfeldimpulse erzeugt, die auf die Membran 12 einwirken, die abdichtend an den Seitenwänden des verstärkten Kerns 10 befestigt ist, der die Wicklung 11 umschließt. Während der Impulse, deren Folgefrequenz mit Hilfe des Elektroimpulsgenerators geregelt wird, beispielsweise im Bereich von 100 Hz bis 10 kHz, wird die Membran 12 zu dem Elektromagneten des Hydroimpulsmodulators 8 hingezogen. Nach dem Ende des Impulses kehrt die Membran 12 in die Ausgangsposition zurück, wobei sie den Druck der Flüssigkeit moduliert. Diese Vibrationen erzeugen Flüssigkeitsdruckimpulse mit einer Amplitude im Bereich von 20% des statischen Drucks. Der wechselnde Druck wird nur auf den distalen Teil des Dilatationsballons 3 übertragen, da der karkassenartige Ausdehnungsbegrenzer 13 Vibrationen des proximalen Teils des Ballons, in den er eingefügt ist, verhindert. Der karkassenartige Ausdehnungsbegrenzer 13 hat beispielsweise die Form einer Spirale.

Bei dem Vorgang der hin- und hergehenden Vibrationseinwirkung werden bei beiden Varianten der Erfindung Pulse mit geringer Amplitude in Richtung des durch die anfängliche Ausdehnung des Dilatationsballons verdichteten schwammigen Knochengewebes erzeugt. Diese Pulse ermöglichen eine Überwindung der Kompression, die die vollständige Wiederherstellung der Höhe des Wirbelkörpers behindert, sowie ein Richten der Knochensplitter. Gleichzeitig geschieht ein ergänzendes Verdichten des schwammigen Knochengewebes. Mit Hilfe der Sichtkontrolle durch eine Vorrichtung wird der Schritt der Wiederherstellung der Höhe des Wirbelkörpers verfolgt.

Nach dem vollständigen Richten der kyphotischen Deformation wird das Pumpen der Flüssigkeit gestoppt. Der Dilatationsballon 3 wird entleert und aus dem ausgebildeten Hohlraum entfernt. Anschließend wird mittels der Vorrichtung zur Injektion des Knochenersatzmaterials der ausgebildete Hohlraum mit Knochenzement gefüllt. Nach einer gewissen Zeit härtet der Knochenzement aus und ermöglicht die Tragfähigkeit des geschädigten Wirbels.

Im Falle der Verwendung von granuliertem Knochenersatzmaterial wird auf den Handgriff des Trokars das Basisteil mit dem Trichter montiert, in den das granulierte Knochenersatzmaterial geschüttet wird und mittels des Schaftstößels der ausgebildete Hohlraum im Wirbelkörper gefüllt wird. Um den Hohlraum dichter zu füllen, wird zum Beispiel ein Ultraschallverdichter verwendet.

### Beispiel 1:

Patient S., 44 Jahre, kam in die traumatologische Abteilung mit der Diagnose:
"Geschlossenes einfaches Trauma im Brustwirbelbereich. Kompressionsbruch des Wirbelkörpers ThXII mit einem Keilförmigkeitsindex von 0,7 und einem kyphotischen Deformationswinkel von 10°."

Das Trauma hatte er als Folge eines Sturzes von einem Baum erlitten. 6 Stunden nach Erleiden des Traumas wurde er in eine medizinische Einrichtung eingeliefert. Bei der Untersuchung wurden ein starker Schmerz und eine leichte Kyphotisierung im Brustwirbelbereich festgestellt.

Bei der Einlieferung in die stationäre Abteilung wurde der Patient untersucht:
- Bei der Röntgenuntersuchung wurde ein Kompressionsbruch des Wirbelkörpers ThXII mit einem Keilförmigkeitsindex von 0,7 und einem kyphotischen Deformationswinkel von 10° festgestellt.
- Bei der computertomografischen Untersuchung wurde keine Verschiebung von Knochensplittern zum Wirbelkanal festgestellt.
- Bei der magnetresonanztomografischen Untersuchung wurde keine Verletzung der Integrität des Wirbelkanals und seines Inhalts festgestellt.

Bezüglich des neurologischen Zustands wurden keine motorischen und sensorischen Ausfälle festgestellt. Lokal wurde bei Palpation eine ausgeprägte Empfindlichkeit der Dornfortsätze auf der Höhe ThXII-LI festgestellt.

Am auf die Einweisung in die stationäre Abteilung folgenden Tag wurde an dem Patienten ein chirurgischer Eingriff mit Hilfe der Vorrichtung zur Ballonkyphoplastie des Wirbelkörpers, die als erste Variante dieser Anmeldung angemeldet ist, vorgenommen.

Bei der Operation wurde der Patient in Bauchlage auf den Operationstisch gelegt. Es wurde eine Bearbeitung des Operationsbereichs von der Höhe der Schulter bis zum Kreuzbein durchgeführt und eine Lokalanästhesie mit einer 0,5%-igen Novocain-Lösung schichtweise entsprechend dem vorgesehenen Punktionspfad am Vorsprung der Bogenwurzel des Wirbels ThXII links vorgenommen. Weiterhin wurde der Röhrenleiter unter fluoroskopischer Kontrolle zur Bogenwurzel des Wirbels ThXII eingeführt und installiert, anschließend wurde der Leiter bis zur Grenze des vorderen und des mittleren Drittels des Wirbelkörpers (2/3 des Abstands von der Rückwand und 1/3 von der Vorderwand des Wirbelkörpers) in Richtung zur Mittellinie zu dem Bereich der stärksten Kompression des Wirbelkörpers bewegt. Durch den Röhrenleiter wurde die tragende Katheterröhre in den Wirbelkörper ThXII eingeführt, an deren distalem Ende der abdichtend befestigte Dilatationsballon mit im distalen Teil des Ballons in die Wände eingefügten ferromagnetischen Teilchen einer Größe im Bereich von 150 *µ*m angeordnet war. Unter röntgenologischer Kontrolle wurde der Dilatationsballon durch die Druckflüssigkeit ausgedehnt, wobei eine geringe Wiederherstellung der Höhe des Wirbelkörpers beobachtet wurde. Anschließend wurde der Elektromagnet in der Sagittalebene dorsal angeordnet, so dass seine Symmetrieachse auf den distalen Teil des Dilatationsballons gerichtet war.

Physikalisch-technische Kenngrößen des Elektromagneten:
Resonanzfrequenz, bei der der Elektromagnet arbeitet: 22 kHz;
Amplitude des Betriebsstroms bei Reihenresonanz im Bereich 1 A;
Generator der periodischen Vibrationen, Betriebsspannung: 20-25 V;
Begrenzungswiderstand: 7 Ohm;
Amplitude des Wechselmagnetfelds im Bereich der Einwirkung auf den Ballon: 150 mT; Material des Kerns: Permalloy-Blech mit einer Dicke von 0,1 mm;
Spule: 150 Wicklungen eines PEV-Leiters mit einem Durchmesser von 0,3 mm;
Länge des Elektromagneten: 150 mm;
Durchmesser: 45 mm;
Spitzendurchmesser: 5 mm.

Der Elektromagnet wurde an eine Wechselstromquelle angeschlossen, und es wurde mit dem distalen Teil des Dilatationsballons eine zusätzliche stoß-vibrierende Übertragungswirkung auf die Wände des ausgebildeten Knochenhohlraums ausgeübt, wodurch es gelang, nicht nur eine Wiederherstellung der Höhe des Wirbelkörpers bis zu normalen Abmessungen zu erreichen, sondern auch, eine Verdichtung des Knochengewebes am Umfang des ausgebildeten Hohlraums zu erzielen.

Der Dilatationsballon wurde entleert und zusammen mit der tragenden Katheterröhre aus dem Wirbelkörper entfernt. Durch den Röhrenleiter wurde die Einrichtung zur Injektion des Knochenersatzmaterials in den Wirbelkörper ThXII eingeführt, und der ausgebildete Hohlraum im Wirbelkörper wurde mit dem Knochenersatzmaterial in Form eines feinen Granulats, gemischt mit Eigenblut und Omnipack-Kontrastmittel, mit einem Volumen von 4 cm³ gefüllt. Dabei wurde kein Austritt des Knochenersatzmaterials über die Grenzen des Wirbelkörpers festgestellt.

Bei der Röntgenkontrolle wurde eine dichte Füllung des Hohlraums mit Knochenersatzmaterial festgestellt. Anschließend wurde die tragende Katheterröhre entfernt und ein aseptisches Pflaster auf die Punktionsstelle aufgelegt. Der Patient wurde 2 Stunden nach der Operation in eine vertikale Position gebracht und am folgenden Tag entlassen. Kein schmerzhaftes Syndrom war vorhanden. Auf den Kontroll-Röntgenaufnahmen wurde eine vollständige Wiederherstellung der Höhe des Wirbelkörpers ThXII und eine Korrektur der kyphotischen Deformation festgestellt. Bei einer wiederholten Röntgenuntersuchung nach 6 Monaten wurde kein Verlust der Korrektur festgestellt.

### Beispiel 2

Patientin A., 54 Jahre, kam in die traumatologische Abteilung mit der Diagnose: "Geschossenes einfaches Trauma im Brustwirbelbereich. Kompressionsbruch des Wirbelkörpers LI mit einem Keilförmigkeitsindex von 0,6 und einem kyphotischen Deformationswinkel von 13°."

Das Trauma hatte sie infolge eines Autounfalls erlitten. 3 Stunden nach Erleiden des Traumas wurde sie in eine medizinische Einrichtung eingeliefert. Bei der Untersuchung wurden ein starker Schmerz und eine Kyphotisierung im Brustwirbelbereich festgestellt.

Bei der Einlieferung in die stationäre Abteilung wurde die Patientin untersucht:
- Bei der Röntgenuntersuchung wurde ein Kompressionsbruch des Wirbelkörpers LI mit einem Keilförmigkeitsindex von 0,6 und einem kyphotischen Deformationswinkel von 13° festgestellt.
- Bei der computertomografischen Untersuchung wurde keine Verschiebung von Knochensplittern zum Wirbelkanal festgestellt.
- Bei der magnetresonanztomografischen Untersuchung wurde keine Verletzung der Integrität des Wirbelkanals und seines Inhalts festgestellt.

Bezüglich des neurologischen Zustands wurden keine motorischen und sensorischen Ausfälle festgestellt. Lokal wurde bei Palpation eine ausgeprägte Empfindlichkeit der Dornfortsätze auf der Höhe ThXII-LI-LII festgestellt.

Am auf die Einweisung in die stationäre Abteilung folgenden Tag wurde an der Patientin ein chirurgischer Eingriff mit Hilfe der Vorrichtung zur Ballonkyphoplastie vorgenommen, die als zweite Variante angemeldet ist.

Bei der Operation wurde die Patientin in Bauchlage auf den Operationstisch gelegt. Es wurde eine Bearbeitung des Operationsbereichs von der Höhe der Schulter bis zum Kreuzbein durchgeführt und eine Lokalanästhesie mit einer 0,5%-igen Novocain-Lösung schichtweise entsprechend dem vorgesehenen Punktionspfad am Vorsprung der Bogenwurzel des Wirbels LI links vorgenommen. Weiterhin wurde der Röhrenleiter unter fluoroskopischer Kontrolle zur Bogenwurzel des Wirbels LI eingeführt, anschließend wurde der Leiter bis zur Grenze des vorderen und des mittleren Drittels des Wirbelkörpers (2/3 des Abstands von der Rückwand und 1/3 von der Vorderwand des Wirbelkörpers) in Richtung zur Mittellinie zu dem Bereich der stärksten Kompression des Wirbelkörpers bewegt. Durch den Röhrenleiter wurde die tragende Katheterröhre in den Wirbelkörper LI eingeführt, an deren distalem Ende der abdichtend befestigte Dilatationsballon mit dem in den proximalen Teil eingefügten karkassenartigen Ausdehnungsbegrenzer angeordnet war. Unter röntgenologischer Kontrolle wurde der Dilatationsballon durch die Druckflüssigkeit ausgedehnt, wobei eine geringe Wiederherstellung der Höhe des Wirbelkörpers beobachtet wurde. Anschließend wurde mit Hilfe des an den Elektroimpulsgenerator angeschlossenen Hydroimpulsmodulators mit dem distalen Teil des Dilatationsballons eine zusätzliche stoß-vibrierende Übertragungswirkung auf die Wände des ausgebildeten Knochenhohlraums ausgeübt, wodurch es gelang, nicht nur eine Wiederherstellung der Höhe des Wirbelkörpers bis zu normalen Abmessungen zu erreichen, sondern auch, eine Verdichtung des Knochengewebes am Umfang des ausgebildeten Hohlraums zu erzielen.

Der Dilatationsballon wurde entleert und zusammen mit der tragenden Katheterröhre aus dem Wirbelkörper entfernt. Durch den Röhrenleiter wurde die Einrichtung zur Injektion des Knochenersatzmaterials in den Wirbelkörper LI eingeführt, und der ausgebildete Hohlraum im Wirbelkörper wurde mit dem Knochenersatzmaterial in Form eines feinen Granulats, gemischt mit Eigenblut und Omnipack-Kontrastmittel, mit einem Volumen von 2 cm³ gefüllt. Dabei wurde kein Austritt des Knochenersatzmaterials über die Grenzen des Wirbelkörpers festgestellt.

Bei der Röntgenkontrolle wurde eine dichte Füllung des Hohlraums mit Knochenersatzmaterial festgestellt. Anschließend wurde die tragende Katheterröhre entfernt und ein aseptisches Pflaster auf die Punktionsstelle aufgelegt. Die Patientin wurde 2 Stunden nach der Operation in eine vertikale Position gebracht und am folgenden Tag entlassen. Kein schmerzhaftes Syndrom war vorhanden. Auf den Kontroll-Röntgenaufnahmen wurde eine vollständige Wiederherstellung der Höhe des Wirbelkörpers LI und eine Korrektur der kyphotischen Deformation festgestellt. Bei einer wiederholten Röntgenuntersuchung nach 6 Monaten wurde kein Verlust der Korrektur festgestellt.

## Patentansprüche

1. Vorrichtung zur Ballon-Wirbelkyphoplastie, die eine Einrichtung (4,5) zur Injektion von Knochenersatzmaterial in den Wirbelkörper und eine tragende Katheterröhre (1) mit einem Kanal und einer damit verbundenen Druckflüssigkeitspumpe (2) in einen am distalen Ende der tragenden Katheterröhre (1) abdichtend befestigten Dilatationsballon (3) aus elastischem, dehnbarem Material umfasst, ***dadurch gekennzeichnet,* dass** sie einen Elektromagnet (7) umfasst, der einen Ausgang zu einer Wechselstromquelle aufweist und so ausgebildet ist, dass er am Körper des Patienten entlang der Wirbelsäule angeordnet werden kann, wobei am distalen Teil des Dilatationsballons (3) ferromagnetische Elemente (6) eingefügt sind, wobei der Elektromagnet (7) so ausgebildet ist, dass er ein Wechselmagnetfeld erzeugen und damit auf die ferromagnetischen Elemente (6) einwirken kann, um stoß-vibrierende Übertragungsbewegungen am distalen Teil des Dilatationsballons (3) zu ermöglichen.

2. Vorrichtung zur Ballon-Wirbelkyphoplastie nach Anspruch 1, ***dadurch gekennzeichnet,* dass** sie zusätzlich einen Trokar und einen Mandrin umfasst, wobei die tragende Katheterröhre (1) und der Mandrin koaxial gestreckt innerhalb und entlang des Trokars ausgeführt sind und sie nacheinander in ihn hinein- und aus ihm herausgeführt werden können.

3. Vorrichtung zur Ballon-Wirbelkyphoplastie nach Anspruch 1 oder 2, ***dadurch gekennzeichnet,* dass** der Elektromagnet (7) eine langgestreckte Form hat.

4. Vorrichtung zur Ballon-Wirbelkyphoplastie nach einem der vorhergehenden Ansprüche, ***dadurch gekennzeichnet,* dass** die ferromagnetischen Elemente (6) in Form von Teilchen ausgebildet sind, die in die Wände des distalen Teils des Dilatationsballons (3) eingefügt sind.

5. Vorrichtung zur Ballon-Wirbelkyphoplastie nach einem der Ansprüche 1 bis 3, ***dadurch gekennzeichnet,* dass** die ferromagnetischen Elemente (6) in Form von Fäden ausgebildet sind, die an der Innenseite des distalen Teils des Dilatationsballons (3) angeordnet sind.

6. Vorrichtung zur Ballon-Wirbelkyphoplastie nach einem der vorhergehenden Ansprüche, ***dadurch gekennzeichnet,* dass** der Dilatationsballon (3) zusatzlich eine und/oder mehrere Kammern aufweist

7. Vorrichtung zur Ballon-Wirbelkyphoplastie nach einem der Ansprüche 2 bis 6, ***dadurch gekennzeichnet,* dass** sie zusätzlich eine Einrichtung zum Einführen von granuliertem Knochenersatzmaterial aufweist, die aus einem Basisteil mit einem Trichter und einem Schaftstößel mit Handgriff besteht.

8. Vorrichtung zur Ballon-Wirbelkyphoplastie nach Anspruch 2 und 6, ***dadurch gekennzeichnet,* dass** das Basisteil mit dem Trichter der Einrichtung zum Einführen von granuliertem Knochenersatzmaterial, der Mandrin von der Seite seiner proximalen Kante her und die tragende Katheterröhre (1) im Bereich der Verbindung mit der Flüssigkeitspumpe (2) an die Form des Trokarhandgriffs als Paare Vorsprung-Öffnung angepasst sind, wobei sie dicht mit einander verbunden werden können.

9. Vorrichtung zur Ballon-Wirbelkyphoplastie, die eine Einrichtung (4,5) zur Injektion von Knochenersatzmaterial in den Wirbelkörper, eine tragende Katheterröhre (1) mit einem Kanal und einer damit verbundenen Druckflüssigkeitspumpe (2) in einen am distalen Ende der tragenden Katheterröhre (1) abdichtend befestigten Dilatationsballon (3) aus elastischem, dehnbarem Material und mit einem in den proximalen Teil des Dilatationsballons (3) eingefügten karkassenartigen Ausdehnungsbegrenzer (13) des Ballons aus nicht dehnbarem Material umfasst, ***dadurch gekennzeichnet,* dass** darin die Flüssigkeitspumpe (2) einen Hydroimpulsmodulator (8) aufweist, der einen Ausgang zu einem Elektroimpulsgenerator aufweist, wobei der Hydroimpulsmodulator (8) zur Erzeugung von Hydroimpulsen, die auf den Dilatationsballon (3) einwirken, und zur Erzeugung von stoß-vibrierenden Übertragungsbewegungen in dessen distalem Teil ausgebildet ist.

10. Vorrichtung zur Ballon-Wirbelkyphoplastie nach Anspruch 9, ***dadurch gekennzeichnet,* dass** der Hydroimpulsmodulator (9) aus einem von der distalen Seite mit einer flexiblen Membran (12) aus ferromagnetischem Material umschlossenen Elektromagneten besteht, der eine Wicklung (11) und einen verstärkten Kern (19) umfasst, der die Wicklung (11) vollständig umschließt.

## Claims

1. A device for vertebral balloon kyphoplasty comprising means (4, 5) for injecting bone replacement material into the vertebral body and a supporting catheter tube (1) having a conduit and a pressure liquid pump (2) connected thereto into a dilatation balloon (3) made of an elastic, expandable material and being sealingly attached to the distal end of the supporting catheter tube (1),
**characterized in that** it comprises an electromagnet (7) having an outlet to an alternating current source and being configured to be arrangeable at the body of a patient along the vertebral column, wherein ferromagnetic elements (6) are inserted at the distal part of the dilatation balloon (3), wherein the electromagnet (7) is configured to be able to generate an alternating magnetic field and to act on the ferromagnetic elements (6) therewith in order to enable impact-vibration transmission movements at the distal part of the dilatation balloon (3).

2. The device for vertebral balloon kyphoplasty according to claim 1, **characterized in that** it additionally comprises a trocar and a mandrin, wherein the supporting catheter tube (1) and the mandrin are designed to be coaxially extended within and along the trocar and are capable of being successively guided into the trocar and out of the trocar.

3. The device for vertebral balloon kyphoplasty according to claim 1 or 2, **characterized in that** the electromagnet (7) has an elongated shape.

4. The device for vertebral balloon kyphoplasty according to one of the preceding claims, **characterized in that** the ferromagnetic elements (6) are configured in the form of particles inserted into the walls of the distal part of the dilatation balloon (3).

5. The device for vertebral balloon kyphoplasty according to one of claims 1 to 3, **characterized in that** the ferromagnetic elements (6) are configured in the form of filaments arranged at the inside of the distal part of the dilatation balloon (3).

6. The device for vertebral balloon kyphoplasty according to one of the preceding claims, **characterized in that** the dilatation balloon (3) additionally includes one and/or several chambers.

7. The device for vertebral balloon kyphoplasty according to one of claims 2 to 6, **characterized in that** it additionally includes means for introducing granulated bone replacement material consisting of a base part with a funnel and a pusher shaft with a handle.

8. The device for vertebral balloon kyphoplasty according to claim 2 and 6, **characterized in that** the base part with the funnel of the means for introducing granulated bone replacement material, the mandrin from the side of its proximal edge and the supporting catheter tube (1) in the region of its connection to the liquid pump (2) are conformed to the shape of the trocar handle as projection-aperture pairs, wherein they are capable of being sealingly interconnected.

9. A device for vertebral balloon kyphoplasty comprising means (4, 5) for injecting bone replacement material into the vertebral body, a supporting catheter tube (1) having a conduit and a pressure liquid pump (2) connected thereto into a dilatation balloon (3) made of an elastic, expandable material and being sealingly attached to the distal end of the supporting catheter tube (1) and a carcass-like extension limiter (13) of the balloon made of non-expandable material and being inserted into the proximal part of the dilatation balloon (3), **characterized in that** therein that liquid pump (2) includes a hydropulse modulator (8) having an outlet to an electropulse generator, wherein the hydropulse modulator (8) is configured to generate hydropulses acting on the dilatation balloon (3) and to generate impact-vibration transmission movements in the distal part thereof.

10. The device for vertebral balloon kyphoplasty according to claim 9, **characterized in that** the hydropulse modulator (8) consists of an electromagnet enclosed from the distal side by a flexible membrane (12) made of a ferromagnetic material and comprising a winding (11) and a reinforced core (19) completely enclosing the winding (11).

## Revendications

1. Dispositif de cyphoplastie vertébrale à ballonnet qui comprend un agencement (4, 5) pour l'injection de matériau de substitution osseuse dans le corps vertébral et un tube porteur de cathéter (1) comprenant un canal et une pompe de liquide sous pression (2), reliée au canal, disposée dans un ballonnet de dilatation (3) fixé de manière étanchéifiante sur l'extrémité distale du tube porteur de cathéter (1)et constitué d'une matière élastique extensible, ***caractérisé en ce* qu'**il comprend un électroaimant (7) qui présente une sortie vers une source de courant alternatif et est réalisé de manière à pouvoir être disposé sur le corps du patient le long de la colonne vertébrale, des éléments ferromagnétiques (6) étant insérés sur la partie distale du ballonnet de dilatation (3), l'électroaimant (7) étant réalisé de manière à pouvoir générer un champ magnétique alternant et agir ainsi sur les éléments ferromagnétiques (6) afin de permettre des mouvements de transmission vibrant par à-coups sur la partie distale du ballonnet de dilatation (3).

2. Dispositif de cyphoplastie vertébrale à ballonnet selon la revendication 1, ***caractérisé en* ce qu'**il comprend en plus un trocart et un mandrin, le tube porteur de cathéter (1) et le mandrin étant réalisés de manière coaxialement allongés à l'intérieur et le long du trocart et pouvant être guidés l'un après l'autre en y étant enfoncés et y étant ressortis.

3. Dispositif de cyphoplastie vertébrale à ballonnet selon la revendication 1 ou 2, ***caractérisé en ce que*** l'électroaimant (7) a une forme allongée.

4. Dispositif de cyphoplastie vertébrale à ballonnet selon l'une quelconque des revendications précédentes, ***caractérisé en ce que*** les éléments ferromagnétiques (6) sont réalisés en forme de particules qui sont insérées dans les parois de la partie distale du ballonnet de dilatation (3).

5. Dispositif de cyphoplastie vertébrale à ballonnet selon l'une quelconque des revendications 1 à 3, ***caractérisé en ce que*** les éléments ferromagnétiques (6) sont réalisés en forme de fils qui sont disposés sur le côté intérieur de la partie distale du ballonnet de dilatation (3).

6. Dispositif de cyphoplastie vertébrale à ballonnet selon l'une quelconque des revendications précédentes, ***caractérisé en ce que*** le ballonnet de dilatation (3) présente en plus une et/ou plusieurs chambres.

7. Dispositif de cyphoplastie vertébrale à ballonnet selon l'une quelconque des revendications 2 à 6, ***caractérisé en* ce qu'**il présente en plus un dispositif destiné à introduire du matériau granulé de substitution osseuse, lequel dispositif est constitué d'une pièce de base comprenant un entonnoir et un coulisseau à tige avec poignée.

8. Dispositif de cyphoplastie vertébrale à ballonnet selon les revendications 2 et 6, ***caractérisé en ce* que** la pièce de base comprenant l'entonnoir est adaptée au dispositif destiné à introduire du matériau granulé de substitution osseuse, **en ce que** le mandrin, depuis le côté de son bord proximal, et le tube porteur de cathéter (1), dans la partie de liaison avec la pompe de liquide (2), sont adaptés à la forme de la poignée de trocart en tant que paire d'ouverture en saillie, ceux-ci pouvant être reliés entre eux de manière étanche.

9. Dispositif de cyphoplastie vertébrale à ballonnet qui comprend un agencement (4, 5) pour l'injection de matériau de substitution osseuse dans le corps vertébral et un tube porteur de cathéter (1) comprenant un canal et une pompe de liquide sous pression (2), reliée au canal, disposée dans un ballonnet de dilatation (3) fixé de manière étanchéifiante sur l'extrémité distale du tube porteur de cathéter (1) et constitué d'une matière élastique extensible et comprenant un délimiteur d'extension (13) du ballonnet en forme de carcasse, inséré dans la partie proximale du ballonnet de dilatation (3)et constitué de matière non extensible, *caractérisé ce que* la pompe de liquide (2) présente un modulateur d'hydro-impulsions (8) qui présente une sortie vers un générateur d'impulsions électriques, le modulateur d'hydro-impulsions (8) étant réalisé pour générer des hydro-impulsions agissant sur le ballonnet de dilatation (3)et pour générer des mouvements de transmission vibrant par à-coups dans son extrémité distale.

10. Dispositif de cyphoplastie vertébrale à ballonnet selon la revendication 9, ***caractérisé en* ce que** le modulateur d'hydro-impulsions (9) est constitué d'un électroaimant entouré du côté distal d'une membrane flexible (12) en matériau ferromagnétique, lequel électroaimant comprend un enroulement (11) et un noyau renforcé (19) qui entoure l'enroulement (11) complètement.
